# EUROPEAN PATENT APPLICATION

(11) **EP 3 138 419 A1**
(43) Date of publication of application: **08.03.2017**
(21) Application number: 14890718.1
(22) Date of filing: 30.04.2014
(51) Int. Cl.: A24F 47/00

(54) **ELECTRONIC CIGARETTE AND ELECTRONIC CIGARETTE OPERATING METHOD**

(71) Applicant: Huizhou Kimree Technology Co., Ltd. Shenzhen Branch, Guangdong 518000 (CN)
(72) Inventor: XIANG, Zhiyong, Huizhou Guangdong 516000 (CN)
(74) Representative: Studio Torta S.p.A.
(86) International application number: PCT/CN2014/076583
(87) International publication number: WO 2015/165066

(57) **Abstract**

An electronic cigarette playing different melodies according to different amounts of smoking air flow, and an electronic cigarette operating method; the electronic cigarette comprises an electronic cigarette body (100); the electronic cigarette body (100) is provided with an air flow sensor (102), an atomization assembly (103), a sound module (104), and a microcontroller (101) for receiving and identifying different pulse signals and transmitting control signals according to different pulse signals; when a user smokes, the air flow sensor (102) generates pulse signals according to the air flow rate in the electronic cigarette body (100), and the microcontroller (101) identifies the pulse signals and transmits the corresponding control signals to the sound module (104), such that the sound module (104) generates different sounds according to different air flow rates, and the atomization assembly (103) simultaneously atomizes the cigarette liquid to produce smoke. When smoking, the user hears different melodies according to different smoking air flows, thus cultivating the taste of the user, and making the user feel content. The method of adjusting melodies based on the smoking air flow skillfully guides a smoker to utilize the electronic cigarette, being helpful to the physical and mental health of the smoker, and facilitating quitting smoking.

## Description

### FIELD

The present disclosure relates to the field of electronic cigarette, and in particular to an electronic cigarette and a melody playing method playing different melodies based on different smoking airflow rates.

### BACKGROUND

An electronic cigarette is a new type of electronic products. The electronic cigarette has a similar appearance and a similar taste as the ordinary cigarette, but the electronic cigarette is healthier and more environmental friendly than a traditional cigarette.

An electronic cigarette in the conventional technology includes a battery rod component and an atomization component, the battery rod component is connected to one end of the atomization component, and the other end of the atomization component is connected to a suction nozzle. When a user smokes, an airflow sensor may sense actions of the user's smoking, and a controller in the electronic cigarette may control a heating wire component of the atomization component to atomize cigarette liquid to generate vapor.

Existing electronic cigarettes do not make any sound, when a user smokes. Even if there is a sound, the sound is only to imitate a burning sound of smoking cigarette. The voice is depressing and simple without fun, which are not good for the physical and mental health of a smoker, and the effect of smoking cessation is bad.

### SUMMARY

An electronic cigarette and a melody playing method playing different melodies based on different smoking airflow rates are provided according to the disclosure.

An electronic cigarette is provided by the present disclosure, which includes an electronic cigarette body. An airflow sensor, an atomization component, a voice module and a microcontroller are arranged in the electronic cigarette body, and the microcontroller is configured to receive and identify different pulse signals and transmit a control signal based on the different pulse signals. The airflow sensor is connected to the microcontroller and is configured to transmit the different pulse signals to the microcontroller based on different flow rates of airflow in the electronic cigarette body. The voice module is connected to the microcontroller and is configured to receive the control signal and send a melody. The atomization component is connected to the microcontroller and is configured to atomize cigarette liquid to generate vapor.

The electronic cigarette body further includes an atomization control module, and the atomization control module is connected to the microcontroller and is configured to turn on or turn off the atomization component.

The electronic cigarette body further includes a switch module, and the switch module is connected to the microcontroller and is configured to turn on or turn off the voice module.

The electronic cigarette body further includes a voice adjustment module, and the voice adjustment module is connected to the microcontroller and is configured to change the melody of the voice module.

The electronic cigarette body further includes a voice recording module, and the voice recording module is connected to the microcontroller and is configured to record a melody.

The voice recording module includes a receiving unit and a button unit, and the receiving unit and the button unit are connected to the microcontroller and are configured to control the voice recording module to receive a melody command.

The electronic cigarette body further includes an over-current protection module, and the over-current protection module is connected to the microcontroller and is configured to protect a circuit from being short-circuited.

The electronic cigarette body further includes a display device, and the display device is connected to the microcontroller and is configured to display an operating state of the electronic cigarette.

The display device is an LED light or a display screen.

The voice module is a loudspeaker or a buzzer.

The airflow sensor includes a cylindrical housing, the microcontroller is arranged on a first end of the housing, a baffle plate is arranged on a second end of the housing, and a negative pressure chamber is arranged in the housing. A sensor connected to the microcontroller is arranged in the negative pressure chamber, and the sensor is configured to generate the pulse signal in a case that it is detected that pressure in the negative pressure chamber decreases.

A through-hole is arranged in the baffle plate, a hollow metal rod is arranged in the through-hole, and an annular insulation sleeve is arranged at a position on the bottom of the metal rod corresponding to the through-hole, and the annular insulation sleeve is configured to insulate the baffle plate from the rod.

An electronic cigarette operating method for playing different melodies based on different smoking airflow rates is provided according to the present disclosure. The method is applied to the above electronic cigarette and the method includes: transmitting, by the airflow sensor, a first pulse signal to the microcontroller based on a flow rate of airflow in the electronic cigarette body; transmitting, by the microcontroller, a first control signal to the voice module based on the first pulse signal; and sending, by the voice module, a melody based on the first control signal.

The method further includes: transmitting, by the microcontroller, a second control signal to the atomization component based on the first pulse signal; and atomizing, by the atomization component, cigarette liquid to generate vapor based on the second control signal.

The method further includes: transmitting, by the microcontroller, a third control signal to a display module based on the first pulse signal; and displaying, by the display module, an operating state of the electronic cigarette based on the third control signal.

The method further includes: transmitting, by an atomization control module, a second pulse signal to the microcontroller; transmitting, by the airflow sensor, the first pulse signal to the microcontroller based on the flow rate of the airflow in the electronic cigarette body; and transmitting, by the microcontroller, the first control signal to the voice module based on the pulse signals.

It can be seen from the above technical solutions that the present disclosure has the following advantages.

In the present disclosure, when a user smokes, an airflow sensor generates a pulse signal based on a flow rate of airflow in an electronic cigarette body, a microcontroller in the electronic cigarette body identifies the pulse signal and transmits a corresponding control signal to a voice module, and an atomization component atomizes cigarette liquid to generate smoke. In this way, when smoking an electronic cigarette, the user can hear different melodies based on different smoking airflow rates, which can cultivate the taste of the user and make the user feel pleasant during smoking. The method of adjusting melodies based on a smoking airflow rate can skillfully guides a smoker to smoke the electronic cigarette, thereby, being good to the physical and mental health of the smoker, and making the effect of smoking cessation better.

### BRIEF DESCRIPTION OF THE DRAWINGS

To illustrate technical solutions according to embodiments of the present disclosure or in the conventional technologies more clearly, drawings to be used in the descriptions of the conventional technologies or the embodiments are described briefly hereinafter. Apparently, the drawings described hereinafter are only for some embodiments of the present disclosure, and other drawings may be obtained by those skilled in the art based on those drawings without creative efforts.
Figure 1 is an overall schematic diagram of an electronic cigarette according to an embodiment of the present disclosure;
Figure 2 is a schematic diagram of an electronic cigarette according to an embodiment of the present disclosure;
Figure 3 is a schematic diagram of an electronic cigarette according to another embodiment of the present disclosure;
Figure 4 is a sectional structure schematic diagram of an airflow sensor of an electronic cigarette according to an embodiment of the present disclosure;
Figure 5 is a schematic diagram of a circuit connection of an electronic cigarette according to another embodiment of the present disclosure; and
Figure 6 is a schematic diagram of an operating method of an electronic cigarette for playing different melodies based on different smoking airflow rates according to an embodiment of the present disclosure.

### DETAILED DESCRIPTION

Hereinafter technical solutions of embodiments of the present disclosure are illustrated clearly and completely in conjunction with drawings of the embodiments of the present disclosure. Apparently, the described embodiments are merely a few rather than all of the embodiments of the present disclosure. All other embodiments obtained by persons of ordinary skill in the art based on the embodiments of the present disclosure without creative efforts shall fall within the protection scope of the present disclosure.

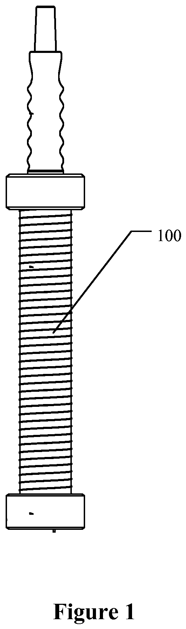

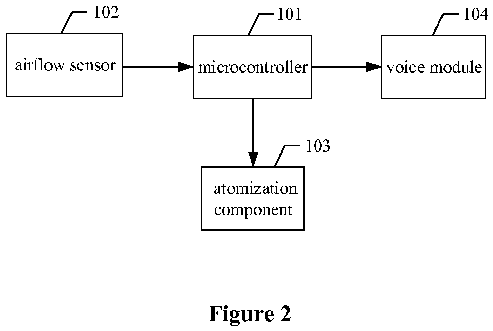

An electronic cigarette playing different melodies based on different smoking airflow rates is provided according to the present disclosure. A specific structure of the electronic cigarette refers to Figure 1 and Figure 2. The electronic cigarette includes an electronic cigarette body 100. An airflow sensor 102, an atomization component 103, a voice module 104 and a microcontroller 101 are arranged in the electronic cigarette body, and the microcontroller 101 is configured to receive and identify different pulse signals and transmit a control signal based on the different pulse signals. The airflow sensor 102 is connected to the microcontroller 101 and is configured to transmit the different pulse signals to the microcontroller 101 based on different flow rates of airflow in the electronic cigarette body 100. The voice module 104 is connected to the microcontroller 101 and is configured to receive the control signal and send a melody. And the atomization component 103 is connected to the microcontroller 101 and is configured to atomize cigarette liquid to generate vapor.

In the embodiment of the present disclosure, the airflow sensor 102, the atomization component 103, the voice module 104 and the microcontroller 101 are arranged in the electronic cigarette body.

When a user smokes, if the airflow sensor 102 detects a change of air pressure in the electronic cigarette body 100, the airflow sensor 102 transmits different pulse signals based on different airflow rates. That is, the airflow sensor 102 is provided with a sensor which can detect a flow rate of airflow in the electronic cigarette body 100, and transmits a pulse to the microcontroller 101, after the flow rate of airflow is detected. A sensing element of the airflow sensor 102 classify the flow rate of airflow into different grades, the flow rates of airflow within a certain range is set for each grade, and each flow rate grade corresponds to a different pulse signal transmitted by the airflow sensor 102. For example, when a user smokes, air pressure reaches a stable value after 1.2 seconds, that is, a flow rate of airflow in the electronic cigarette body 100 reaches a constant value, the airflow sensor 102 detects the constant value, and the microcontroller 101 transmits a pulse signal corresponding to the constant value. The example is intent to better illustrate an implementation manner of the embodiment, which is not limited.

The microcontroller 101 receives the pulse signal and transmits a corresponding control signal based on the received pulse information. The control signal corresponds to the pulse signal. The microcontroller 101 transmits the control signal to the voice module 104. The voice module 104 are provided with several different melodies, the voice module 104 controls the different melodies to send a corresponding melody based on the control signal transmitted by the microcontroller 101.

The microcontroller 101 transmits a control signal to the atomization component 103 based on the pulse signal. The atomization component 103 atomizes cigarette liquid in the electronic cigarette body to generate vapor based on the control signal.

In this way, a user may hear a melodious melody, while smoking an electronic cigarette, and the user may hear different melodies by controlling different inspiration capacities, which increases the enjoyment of smoking the electronic cigarette, cultivates the taste of the user and makes the user feel pleasant during smoking. The method of adjusting melodies based on a smoking airflow rate can skillfully guides a smoker to smoke the electronic cigarette, thereby, being good to the physical and mental health of the smoker, and making the effect of smoking cessation better.

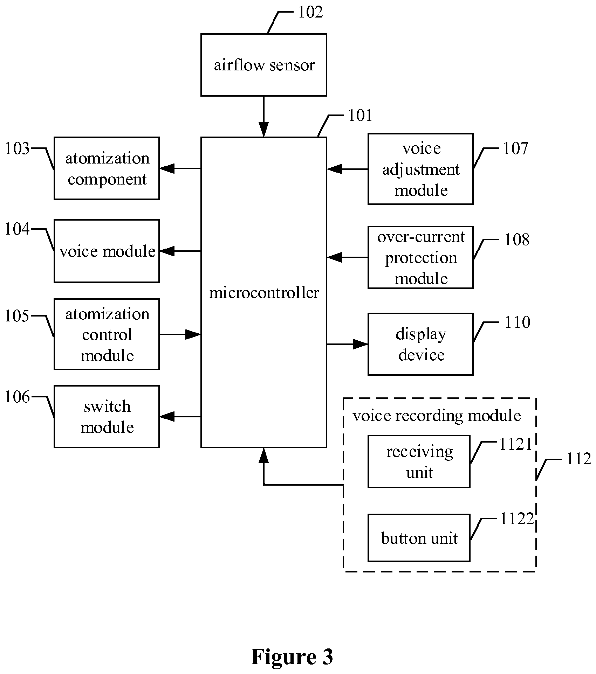

In the embodiment, in order to increase more usage functions and bring better experience to a user, some functional components are added in the electronic cigarette body. Reference is made to Figure 3. One item of the functional components may be added alone, or several items of the functional components may be added, or all of the functional components may be included, which are not limited. Hereinafter usage modes and principles of every functional component are described.

In the embodiment, the electronic cigarette body 100 further includes an atomization control module 105, and the atomization control module 105 is connected to the microcontroller 101 and is configured to turn on or turn off the atomization component 103.

The atomization control module 105 may be installed outside the electronic cigarette body in a form of button switch or change-over switch, which is not specific limited. A user may turn on or turn off the atomization component 103 by using the atomization control module 105. In a case that the user turns off the atomization component 103, the user only hears a melody when smoking an electronic cigarette, that is, only the voice module 104 operates and plays the melody, and the atomization component 103 does not operate.

In the embodiment, the electronic cigarette body 100 further includes a switch module 106, and the switch module 106 is connected to the microcontroller 101 and is configured to turn on or turn off the voice module 104.

The switch module 106 may be installed outside the electronic cigarette body in a form of button switch or change-over switch, which is not specific limited. The user may turn on or turn off the voice module 104 by using the switch module 106. When the user smokes, the atomization component 103 operates to generate vapor, and the voice module 104 does not operate, so the user can not hear a melody.

In the embodiment, the electronic cigarette body 100 further includes a voice adjustment module 107, and the voice adjustment module 107 is connected to the microcontroller 101 and is configured to change the melody of the voice module 104. The switch module 106 may be installed outside the electronic cigarette body in a form of button switch or change-over switch, which is not specific limited. The user may select a playing order of melodies as the user likes via the voice adjustment module 107, that is, a flow rate of airflow when a user smokes corresponding to the voice module 104 is changed via the voice adjustment module 107. A user may generate auditory fatigue, if the electronic cigarette always plays melodies in a certain order.

In the embodiment, the electronic cigarette body 100 further includes a voice recording module 112, and the voice recording module 112 is connected to the microcontroller 101 and is configured to record a melody.

The voice recording module 112 includes a receiving unit 1121 and a button unit 1122, and the receiving unit 1121 and the button unit 1122 are connected to the microcontroller 101 and are configured to control the voice recording module 112 to receive a melody command.

The button unit 1122 may be installed outside the electronic cigarette body in a form of button switch or change-over switch, which is not specific limited. A user inputs a melody that the user likes into the microcontroller 101 by using the button unit 1122 and via the receiving unit 1121, such as, music, sound of animals and ringtones. A storage cell is arranged in the microcontroller 101 and is configured to store melodies inputted by the user. The user changes and sets the melody played by the voice module 104 via the voice adjustment module 107, which makes the electronic cigarette more interesting and humanized.

In the embodiment, the electronic cigarette body 100 further includes an over-current protection module 108, and the over-current protection module 108 is connected to the microcontroller 101 and is configured to protect a circuit from being short-circuited.

To protect circuits in the embodiment, the over-current protection module 108 is used to detect a current of the circuit. When a short circuit occurs in the circuit, the over-current protection module 108 turns off a power loop, which can protect electronic components in the circuit. The over-current protection module 108 may be a fuse or a fusible switch, which is not limited.

In the embodiment, the electronic cigarette body 100 further includes a display device 110, and the display device 110 is connected to the microcontroller 101 and is configured to display an operating state of the electronic cigarette. The display device 110 is an LED light or a display screen.

In order to make a user more understand the operating state of the electronic cigarette, the display device 110 may be arranged outside the electronic cigarette body. An example of LED light is taken, and the example is not limited. Several LED lights of different colors are arranged outside the electronic cigarette body. When the user smokes an electronic cigarette, shining of a red LED light shows that the atomization component 103 of the electronic cigarette is operating, and shining of a yellow LED light shows that the current voice module 104 is playing a melody corresponding to the yellow LED light. Color configuration of LED lights may correspond to a playing order of the melodies played by the voice module 104.

In the embodiment, a battery component is arranged in the electronic cigarette body 100, and a shortage of power is reminded via flickering of an LED light, when the battery component is out of power.

In the embodiment, the voice module 104 is a loudspeaker or a buzzer, which is not limited.

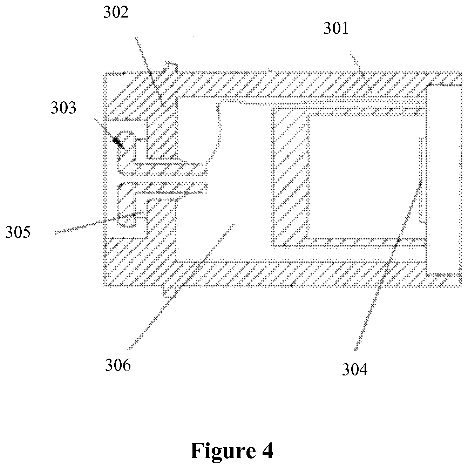

In the embodiment, a detailed structure of the airflow sensor 102 is shown in Figure 4. Specifically, the airflow sensing switch 102 includes a cylindrical housing 301. The microcontroller 101 is arranged on the housing 301, and a baffle plate 302 is arranged on a second end of the housing 301. A through-hole is arranged in the baffle plate 302, and a hollow metal rod 303 is arranged in the through-hole.

An annular insulation sleeve 305 is arranged at a position on the bottom of the metal rod 303 corresponding to the through-hole, and the annular insulation sleeve 305 is configured to insulate the baffle plate 302 from the rod 303.

A negative pressure chamber 306 is arranged in the housing 301. A sensor 304 connected to the microcontroller 101 is arranged in the negative pressure chamber 306, and the sensor 304 is configured to generate the pulse signal in a case that it is detected that pressure in the negative pressure chamber 306 decreases.

Specifically, the microcontroller is located inside or outside the negative pressure chamber 306. A specific location is not limited in the embodiment.

In the embodiment, a specific application process of the airflow sensor 102 is described as below. When a user smokes, if pressure of the negative pressure chamber 306 in the cylindrical housing 301 decreases, airflow may generate in the negative pressure chamber 306, the airflow sensor 304 senses the case that the pressure of the negative pressure chamber 306 decreases, generates a pulse signal, and transmits the pulse signal to the microcontroller 101.

And the microcontroller 101 outputs a control signal to turn on a switch of the circuit in which the atomization component 103 locates, and an atomizer operates to generate vapor. A switch of the circuit in which the voice module 104 locates is turned on, and the voice module 104 plays a melody. When the user stops smoking, the microcontroller 101 can not detect the pulse signal and will turn off the switches of the circuits in which the atomization component 103 and the voice module 104 locate to cause the atomization component 103 and the voice module 104 to stop operating.

The airflow sensor 304 does not generate any signal, when the pressure in the negative pressure chamber 306 increases or the negative pressure chamber 306 shakes due to an impact of external forces. That is to say, the pulse signal is generated only when a user smokes and the negative pressure chamber 306 produces negative pressure.

The microcontroller 101 and the housing 301 have a good sealing to ensure a good sealing of the negative pressure chamber 306. And the negative pressure chamber 306 maintains connection in air with the outside only through the hollow metal rod 302.

In the embodiment, the airflow sensor 102 may sense a change of the pressure in the negative pressure chamber 306, when a user smokes. Therefore, in a case that the pressure in the negative pressure chamber 306 decreases, different pulse signals are generated, and the pulse signals make the microcontroller turn on the switch of the circuit in which the atomizer locates, to enable the atomizer to operate normally to generate vapor, thereby, enabling a smoking process of the user to be more real. And the microcontroller 101 controls the voice module 104 to play a melody based on the pulse signal.

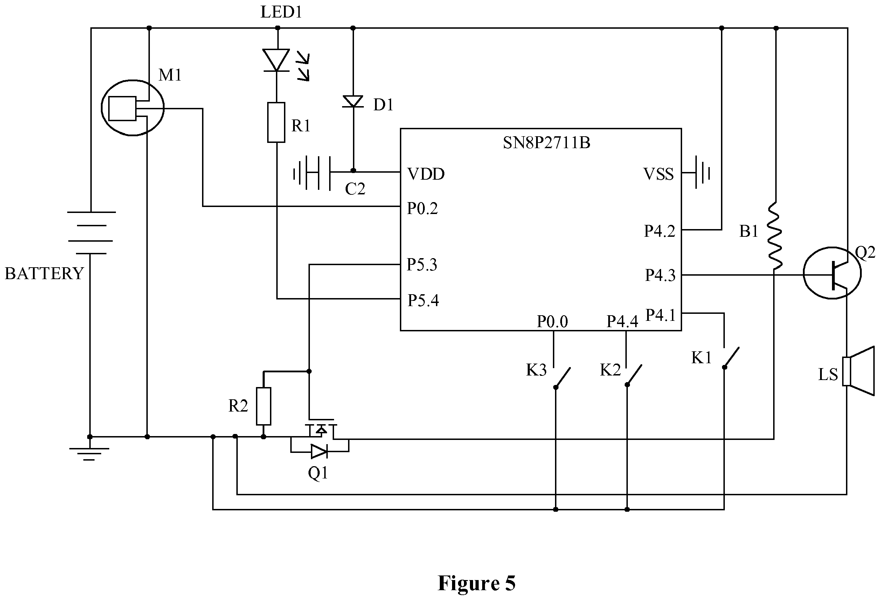

In order to better understand the embodiment, referring to Figure 5, a specific circuit diagram is described to illustrate an implementation of the embodiment. The circuit diagram is only for complete description and is not intended to limit the disclosure.

As shown in Figure 5, specific components are listed. M1 is an airflow sensor 102, a specific type of which may be selected as UAS1000, a specific type of a microcontroller 101 may be selected as SN8P2711B, Q2 is a triode, LS is a voice module 104, B1 is an electric heating wire of an atomization component 103, K1 is a voice adjustment module 107, K2 is a switch module 106, and K3 is an atomization control module 105.

A detailed operating process of the circuit is described as below. When a user smokes, airflow passes the airflow sensor 102, the airflow sensor 102 measures an rate of the airflow and transmits the rate to the microcontroller 101, the microcontroller 101 controls the voice module 104 to send different sounds based on the received different signals of rates of the airflow. A field effect transistor Q1 in a switch component is turned on, to enable a battery BATTERY to supply power to the electric heating wire B1 of the atomization component 103 so that the electric heating wire B1 atomizes cigarette liquid. And LED1 configured to imitate a cigarette smoking is controlled to be lighted. Therefore, it is achieved that the user may hear melodious music, while smoking an electronic cigarette.

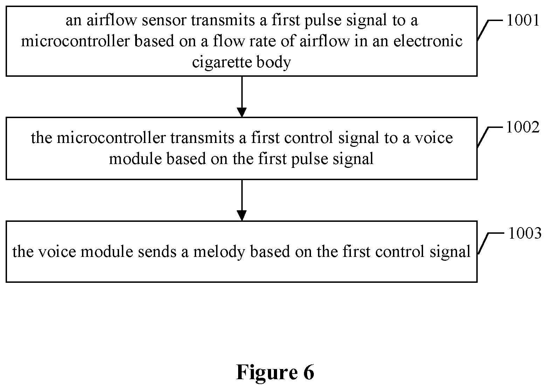

An electronic cigarette operating method for playing different melodies based on different smoking airflow rates is provided according to an embodiment. Reference is made to Figure 6. The method is applied to the electronic cigarette in the above embodiment and the method includes steps 1001 to 1003.

In step 1001, the airflow sensor 102 transmits a first pulse signal to the microcontroller 101 based on a flow rate of airflow in the electronic cigarette body.

When a user smokes, the airflow sensor 102 detects a change of air pressure in the electronic cigarette body 100 and transmits different pulse signals based on different airflow rates. That is to say, the airflow sensor 102 is provided with a sensor which can detect the flow rate of airflow of the electronic cigarette body 100, and the airflow sensor 102 transmits the first pulse signal to the microcontroller 101, after the sensor detects the flow rate of airflow.

In step 1002, the microcontroller 101 transmits a first control signal to the voice module 104 based on the first pulse signal.

After receiving the first pulse signal, the microcontroller 101 transmits the first control signal to the voice module 104, to enable the voice module 104 to be ready to operate.

In step 1003, the voice module 104 sends a melody based on the first control signal.

The microcontroller 101 transmits the first control signal to the voice module 104. Several different melodies have been set in the voice module 104, and the voice module 104 having the different melodies sends a corresponding melody based on the first control signal transmitted by the microcontroller 101.

In the embodiment, the method further includes: transmitting, by the microcontroller 101, a second control signal to the atomization component 103 based on the first pulse signal; and atomizing, by the atomization component 103, cigarette liquid to generate vapor based on the second control signal.

The microcontroller 101 transmits the second control signal to the atomization component 103 based on the pulse signal, while transmitting the first control signal to the voice module 104. The atomization component 103 atomizes the cigarette liquid in the electronic cigarette body to generate vapor based on the second control signal.

In this way, a user may hear a melodious melody, while smoking an electronic cigarette. In addition, the user may hear different melodies by controlling different inspiration capacities, which increases the enjoyment of smoking the electronic cigarette.

In the embodiment, the method further includes: transmitting, by the microcontroller 101, a third control signal to a display module 110 based on the first pulse signal; and displaying, by the display module 110, an operating state of the electronic cigarette based on the third control signal.

In order to make an electronic cigarette more realistic and make a user smoke the electronic cigarette like smoking a cigarette, in the embodiment, the display module 110 is arranged on one end of the electronic cigarette. When a user smokes an electronic cigarette to generate vapor and hears a melody, the display module 110 on one end of the electronic cigarette imitates a cigarette smoking by using LED lights or other light-emitting elements, which bring more realistic feels to the user.

In the embodiment, the method further includes: transmitting, by an atomization control module 105, a second pulse signal to the microcontroller 101; transmitting, by an airflow sensor 102, the first pulse signal to the microcontroller 101 based on the flow rate of the airflow in the electronic cigarette body 100; and transmitting, by the microcontroller 101, the first control signal to the voice module 104 based on the pulse signals.

In the embodiment, a user may turn off the atomization component 103 and only hear a melody when smoking. That is, only the voice module 104 operates to plays the melody, and the atomization component 103 does not operate.

Various embodiments in the specification are described in a progressive manner, the differences from other embodiments are emphatically illustrated in each embodiment, and reference can be made to other embodiments for understanding the same or similar parts of the embodiments.

According to the description of the disclosed embodiments, the disclosure may be implemented or used by the person skilled in the art. Various modifications made to these embodiments are apparent for persons skilled in the art, and a normal principle defined in the disclosure may be implemented in other embodiments without departing from spirit or scope of the disclosure. Therefore the disclosure is not limited to the embodiments described in the disclosure but confirms to a widest scope in accordance with principles and novel features disclosed in the disclosure.

## Claims

1. An electronic cigarette, comprising:
an electronic cigarette body (100); and
an airflow sensor (102), an atomization component (103), a voice module (104) and a microcontroller (101) which are arranged in the electronic cigarette body, wherein
the microcontroller (101) is configured to receive and identify different pulse signals and transmit a control signal based on the different pulse signals,
the airflow sensor (102) is connected to the microcontroller (101) and is configured to transmit the different pulse signals to the microcontroller (101) based on different flow rates of airflow in the electronic cigarette body (100),
the voice module (104) is connected to the microcontroller (101) and is configured to receive the control signal and send a melody, and
the atomization component (103) is connected to the microcontroller (101) and is configured to atomize cigarette liquid to generate vapor.

2. The electronic cigarette according to claim 1, wherein
the electronic cigarette body (100) further comprises an atomization control module (105); and
the atomization control module (105) is connected to the microcontroller (101) and is configured to turn on or turn off the atomization component (103).

3. The electronic cigarette according to claim 1, wherein
the electronic cigarette body (100) further comprises a switch module (106); and
the switch module (106) is connected to the microcontroller (101) and is configured to turn on or turn off the voice module (104).

4. The electronic cigarette according to claim 1, wherein
the electronic cigarette body (100) further comprises a voice adjustment module (107); and
the voice adjustment module (107) is connected to the microcontroller (101) and is configured to change the melody of the voice module (104).

5. The electronic cigarette according to claim 1, wherein
the electronic cigarette body (100) further comprises a voice recording module (112); and
the voice recording module (112) is connected to the microcontroller (101) and is configured to record a melody.

6. The electronic cigarette according to claim 5, wherein
the voice recording module (112) comprises a receiving unit (1121) and a button unit (1122); and
the receiving unit (1121) and the button unit (1122) are connected to the microcontroller (101) and are configured to control the voice recording module to receive a melody command.

7. The electronic cigarette according to claim 1, wherein
the electronic cigarette body (100) further comprises an over-current protection module (108); and
the over-current protection module (108) is connected to the microcontroller (101) and is configured to protect a circuit from being short-circuited.

8. The electronic cigarette according to claim 1, wherein
the electronic cigarette body (100) further comprises a display device (110); and
the display device (110) is connected to the microcontroller (101) and is configured to display an operating state of the electronic cigarette.

9. The electronic cigarette according to claim 8, wherein the display device (110) is an LED light or a display screen.

10. The electronic cigarette according to claim 1, wherein the voice module (104) is a loudspeaker or a buzzer.

11. The electronic cigarette according to claim 1, wherein
the airflow sensor (102) comprises a cylindrical housing (301);
the microcontroller (101) is arranged on a first end of the housing (301);
a baffle plate (302) is arranged on a second end of the housing (301);
a negative pressure chamber (306) is arranged in the housing (301); and
a sensor (304) connected to the microcontroller (101) is arranged in the negative pressure chamber (306), and the sensor (304) is configured to generate the pulse signal in a case that it is detected that pressure in the negative pressure chamber (306) decreases.

12. The electronic cigarette according to claim 11, wherein
a through-hole is arranged in the baffle plate (302);
a hollow metal rod (303) is arranged in the through-hole; and
an annular insulation sleeve (305) is arranged at a position on the bottom of the metal rod (303) corresponding to the through-hole, and the annular insulation sleeve (305) is configured to insulate the baffle plate (302) from the rod (303).

13. An electronic cigarette operating method for playing different melodies based on different smoking airflow rates, wherein the method is applied to the electronic cigarette according to claims 1 to 12, and the method comprises:
transmitting, by the airflow sensor (102), a first pulse signal to the microcontroller (101) based on a flow rate of airflow in the electronic cigarette body;
transmitting, by the microcontroller (101), a first control signal to the voice module (104) based on the first pulse signal; and
sending, by the voice module (104), a melody based on the first control signal.

14. The electronic cigarette operating method according to claim 13, wherein the method further comprises:
transmitting, by the microcontroller (101), a second control signal to the atomization component (103) based on the first pulse signal; and
atomizing, by the atomization component (103), cigarette liquid to generate vapor based on the second control signal.

15. The electronic cigarette operating method according to claim 13, wherein the method further comprises:
transmitting, by the microcontroller (101), a third control signal to a display module (110) based on the first pulse signal; and
displaying, by the display module (110), an operating state of the electronic cigarette based on the third control signal.

16. The electronic cigarette operating method according to claim 13, wherein the method further comprises:
transmitting, by an atomization control module (105), a second pulse signal to the microcontroller (101);
transmitting, by the airflow sensor (102), the first pulse signal to the microcontroller (101) based on the flow rate of the airflow in the electronic cigarette body (100); and
transmitting, by the microcontroller (101), the first control signal to the voice module (104) based on the pulse signals.
